# EUROPÄISCHE PATENTSCHRIFT

(11) **EP 1 139 998 B1**
(45) Veröffentlichungstag und Bekanntmachung des Hinweises auf die Patenterteilung: **02.11.2005**
(21) Anmeldenummer: 99959364.3
(22) Anmeldetag: 30.11.1999
(51) Int. Cl.: A61K 7/42

(54) **VERWENDUNG VON WASSERUNLÖSLICHEN LINEAREN POLY-ALPHA-GLUCAN ALS UV-FILTER**
UTILISATION OF WATER-INSOLUBLE LINEAR POLY-ALPHA-GLUCAN AS UV FILTER
UTILISATION DE POLY-ALPHA-GLUCANE LINEAIRE SOLUBLE DANS L'EAU COMME FILTRE U.V.

(30) Priorität: 28.12.1998 DE 19860370
(43) Veröffentlichungstag der Anmeldung: 10.10.2001
(73) Patentinhaber: Celanese Ventures GmbH, 65926 Frankfurt am Main (DE)
(72) Erfinder: BENGS, Holger, D-60598 Frankfurt (DE); BRAUNAGEL, Alfred, D-55128 Mainz (DE)
(74) Vertreter: Luderschmidt, Schüler & Partner GbR
(86) Internationale Anmeldenummer: PCT/EP1999/009292
(87) Internationale Veröffentlichungsnummer: WO 2000/038645

(56) Entgegenhaltungen:
- WO-A-94/18932
- US-A- 4 894 222
- US-A- 5 256 404
- PATENT ABSTRACTS OF JAPAN vol. 1999, no. 14, 22. Dezember 1999 (1999-12-22) & JP 11 246379 A (KOSE CORP), 14. September 1999 (1999-09-14)
- INABA, R. ET AL.: "Application of porous starch complex powder" STN INTERNATIONAL, FILE CAPLUS, AN=1995:883105, XP002130221 & J.SCCJ (1995), Bd. 29, Nr. 2, Seiten 146-153,
- "THE MERCK INDEX * TWELFTH EDITION" 1996 , MERCK RESEARCH LABORATORIES , NJ XP002131698 Seite 1502, linke Spalte, Absatz 8954

## Beschreibung

Die vorliegende Erfindung betrifft die Verwendung von wasserunlöslichen linearen Poly-alpha-glucan als UV-Filter sowie ein Sonnenschutzmittel enthaltend wasserunlösliches lineares Poly-alpha-glucan, das biotechnisch erzeugt worden ist.

Bekannte UV-Filter sind z.B. Pigmente wie Titandioxid und Zinkoxid als solche oder in Form von sogenannten mikronisierten Partikeln.

Diese Pigmente haben vom kosmetischen Gesichtsichtspunkt aus den Nachteil, daß sie weißeln, d.h. die Haut weiß gefärbt erscheinen lassen.

Als Kompromiß zur Verringerung des Weißelns auf ein erträgliches Maß und dennoch Erzielen eines akzeptablen UV-Schutzes werden diese Pigmente üblicherweise mit einer Teilchengröße zwischen 10 und 100 nm in Sonnenschutzmitteln eingesetzt (A. Schrader, M. Rohr "Auffälligkeiten bei der Entwicklung und Prüfung von mikropigmenthaltigen Sonnenschutzformulierungen" SÖFM-Joumal, 124; Seiten 478-487, 8/98).

Titandioxid ist zudem unter dem gesundheitlichen Aspekt kritisch zu betrachten. In einer Studie mit Titandioxid, das aus Sonnenschutzmittel extrahiert worden ist, wurde bei Bestrahlung mit Sonnenlicht beobachtet, daß Titandioxid Photooxidation zu katalysieren vermag und Bakterien-DNA schädigt (CTFA/TRN Volume 12, No. 3, Seite 5 (1998) mit Verweis auf FEBS Letters, 418, 87-90, 1997).

Eine lichtschützende Wirkung ist auch für einzelne Polysaccharide bekannt. So ist für Poly-β-1,3-Glucan eine lichtschützende Wirkung beschrieben in H. Eggensperger, M. Wilker, "Multiaktiv wirksame Polysaccharide Teil I-Pilzextrakte und Teil II-Pflanzliche Polysaccharide"in SÖFW-Joumal, 123, 8/97, Seiten 542-546 und 12/97, Seiten 838-842.
Poly-β-1,3-Glucane, die aus Hefen erhalten werden können, besitzen eine lineare Struktur mit einem geringen Anteil an β-1,6-Verzweigung.

Weiter wird vorgeschlagen, biotechnisch oder aus marinen Mollusken gewonnenes Glykogen, ein hochverzweigtes Poly-1,4-α-glucan mit Verzweigungen in 6-Position, für Sonnenschutzmittel einzusetzen (M. Pauly, G. "Pauly New Polysaccharides Interest in Care Cosmetology" IN-COSMETICS 1997, Conference Proceedings, Seiten 417-444, Verlag für chemische Industrie, H.Ziolkowsky GmbH,1998).

In der EP-B-0 487 000 wird die Verwendung einer emulsionsförmigen kosmetischen Zusammensetzung mit 15 bis 40 Gew.% einer enzymatisch entzweigten Stärke in Sonnenschutzmittel vorgeschlagen, wobei die enzymatisch abgebaute Stärke ein lineares Poly-1,4-α-glucan mit 15 bis 65 Anhydroglucoseeinheiten ist. Es findet sich jedoch kein Hinweis auf eine mögliche Lichtschutzwirkung der dort verwendeten enzymatisch entzweigten Stärke, vielmehr wird sie als Emulgierhilfsmittel eingesetzt.

Das Patent US-A-5 256 404 beschreibt eine Sonnenschutz zusammensetung, welche modifirierte Stärke, Stärke in Verbindung mit herhömmlichen UV-Filtern oder granuläre Stärke enthält.

In Hinblick auf die Risiken einer intensiven UV-Belastung besteht ein zunehmender Bedarf an geeigneten UV-Filtern, bevorzugt solchen, die UV-Strahlung reflektieren können, die nicht nur einen zuverlässigen Schutz gewähren sondern zudem das äußere Erscheinungsbild nicht beeinträchtigen und damit auch für die tägliche Anwendung geeignet sind.

Überraschend wurde gefunden, daß wasserunlösliches lineares Poly-α-glucan eine sehr gute UV-Schutzfunktion aufweist, zudem beim Auftragen transparent,erscheint und nicht weißelt.

Die vorliegende Erfindung betrifft folglich die Verwendung von wasserunlöslichen linearen Poly-α-glucan als UV-Filter mit einer vor UV- Strahlung schützenden Wirkung.

In einer besonders bevorzugten Ausführungsform betrifft die vorliegende Erfindung ein Sonnenschutzmittel, das als UV-Filter biotechnisch, insbesondere biokatalytisch, erzeugtes, wasserunlösliches lineares Poly-α-glucan enthält.

Prinzipiell kann das wasserunlösliche lineare Poly-α-glucan einer beliebigen kosmetischen oder auch medizinischen Zubereitung zugesetzt werden, für die eine UV-schützende Wirkung erwünscht wird. Insbesondere eignet es sich zur Anwendung in dekorativer Kosmetika, um dieser gleichzeitig eine UV-schützende Wirkung zu verleihen und z.B. der Hautalterung durch intensive tägliche Sonnenbestrahlung vorzubeugen.

Ein bevorzugtes Anwendungsgebiet sind auch Sonnenschutzmittel.
Im folgendem wird die Erfindung am Beispiel von Sonnenschutzmitteln näher beschrieben. Es versteht sich jedoch, daß diese Ausführungen nicht auf Sonnenschutzmittel beschränkt sind, sondern ohne weiteres auf andere Anwendungsgebiete, für die ein UV-Schutz erwünscht ist wie die vorstehend genannte Kosmetik, übertragen werden können.

Erfindungsgemäß hergestellte Sonnenschutzmittel mit wasserunlöslichen linearen Poly-α-glucan können einen ausgezeichneten UV-Schutz gewährleisten und erscheinen auch bei hohen Konzentrationen an Poly-α-glucan transparent.

Für die erfindungsgemäß unter Zusatz von wasserunlöslichen linearen Poly-α-glucanen als UV-Filter hergestellten Sonnenschutzmittel oder Kosmetika kann auf die für derartige Mittel üblichen Formulierungen und Zusätze zurückgegriffen werden. Besonders bevorzugte Grundlagen für Formulierungen sind Emulsionen wie z.B. w/o- oder o/w- Emulsionen, wässerige oder fetthaltige Gele, Hydrogele, Öle, emulgatorfreie Emulsionen, Wachs-Öl-Grundlagen etc..

Beispiele für Anwendungsformen sind Cremes, kompaktcremes, Lotionen, Milchen, Masken, Sprays, Fluide, Puder Salben, Salbengrundlagen etc..

Die erfindungsgemäß hergestellten Sonnenschutzmittel oder Kosmetika können neben dem wasserunlöslichen linearen Poly-α-glucan noch weitere, bekannte UV-Filter enthalten.

Der Anteil an Poly-α-glucan richtet sich nach der verwendeten Grundlage und beträgt üblicherweise von etwa 0,5 bis etwa 20 Gew.%, vorzugsweise etwa 2 bis etwa 15 Gew.%, bezogen auf das Gesamtgewicht des Mittels. Für viele Anwendungen reicht oftmals eine Menge von etwa 10 Gew.% oder weniger.

Eine geringere Menge als 0,5 Gew.% ist für einen UV-Schutz ohne Bedeutung.
Bei Bedarf kann auch eine größere Menge als 20 Gew.% eingesetzt werden, beispielsweise werden für sogenannte "Sun Protection Cream Compacts" Anteile bis zu 70 Gew.% eingemischt.

Es versteht sich, daß die Menge stark von der Zusammensetzung des jeweiligen Mittels abhängt.
Enthält das Mittel weitere UV-Filter oder ist die Grundlage des Mittels an sich gefärbt oder pigmentiert wie z.B. pigmentierte o/w- oder w/o-Emulsionen, so daß eine verringerte UV-Durchlässigkeit von vomeherein gegeben ist, können kleinere Mengen an Poly-α-glucan ausreichend sein.
Für UV-durchlässige Grundlagen, z.B. transparente Grundlagen wie unpigmentierte Emulsionen, Gele oder Öle, werden zweckmäßigerweise größere Mengen Poly-α-glucan zugesetzt.

Der benötigte Menge kann von einem Fachmann mit wenigen Routineversuchen jedoch ohne weiteres von Fall zu Fall bestimmt werden.

Wasserunlösliche lineare Poly-α-glucane im Sinne der vorliegenden Erfindung sind Polysaccharide, die aus Glucanen als monomeren Bausteinen derart aufgebaut sind, daß die einzelnen Bausteine stets in der gleichen Art miteinander verknüpft sind. Jede so definierte Grundeinheit oder Baustein hat genau zwei Verknüpfungen, jeweils eine zu einem anderen Monomer. Davon ausgenommen sind lediglich die beiden Grundeinheiten, die den Anfang bzw. das Ende des Polysaccharids bilden. Diese haben nur eine Verknüpfung zu einem weiteren Monomer und bilden die Endgruppen des linearen Polyglucans.

Besitzt die Grundeinheit drei oder mehr Verknüpfungen, wird von Verzweigung gesprochen. Dabei ergibt sich aus der Anzahl der Hydroxylgruppen pro 100 Grundeinheiten, die nicht am Aufbau des linearen Polymerrückgrats beteiligt sind und die Verzweigungen ausbilden, der sogenannte Verzweigungsgrad.

Erfindungsgemäß weisen die linearen wasserunlöslichen Poly-α-glucane einen Verzweigungsgrad von maximal 0,5 % auf, d.h. sie haben maximal 0,5 Verzweigungen auf 100 Grundeinheiten.

Besonders bevorzugt sind Poly-α-glucane deren Verzweigungsgrad in 6-Position maximal 0,5 %, und in den anderen Positionen, z. B. in 2- bzw. 3-Position, vorzugsweise jeweils maximal 2 % und insbesondere 1 % ist.
Besonders bevorzugt sind auch Poly-α-glucane, deren Verzweigungsgrad in 6-Position kleiner als 0,5 % ist.
Für die Erfindung sind insbesondere Poly-α-glucane geeignet, die keine Verzweigungen aufweisen, bzw. deren Verzweigungsgrad so minimal ist, daß er mit herkömmlichen Methoden nicht mehr nachweisbar ist

Beispiele für bevorzugte wasserunlösliche lineare Poly-α-glucane sind lineare Poly-α-D-glucane, wobei die Art der Verknüpfung unwesentlich ist, solange Linearität im Sinne der Erfindung vorliegt. Ein besonders bevorzugtes Beispiel ist Poly-1,4-alpha-D-glucan.

Für die vorliegende Erfindung beziehen sich die Präfixe "alpha" oder "D" allein auf die Verknüpfungen, die das Polymerrückgrat ausbilden und nicht auf die Verzweigungen.
Unter dem Begriff "wasserunlösliches Poly-α-glucan" werden für die vorliegende Erfindung Verbindungen verstanden, die nach der Definition des Deutschen Arzneimittelbuches (DAB = Deutsches Arzneimittelbuch, Wissenschaftliche Verlagsgesellschaft mbH, Stuttgart, Govi-Verlag, Frankfurt, Auflage, 1987) entsprechend den Klassen 4 bis 7 unter die Kategorien "wenig lösliche", "schwer lösliche", "sehr schwer lösliche" bzw. "praktisch unlösliche" Verbindungen fallen.

Im Fall der erfindungsgemäß verwendeten Polyglucane bedeutet dies, daß mindestens 98 % der eingesetzten Menge, insbesondere mindestens 99,5 %, unter Normalbedingungen (T = 25 °C +/- 20 %, p= 101325 Pascal +/- 20 %) in Wasser unlöslich ist (entsprechend den Klassen 4 bzw. 5).

Für die vorliegende Erfindung sind schwer lösliche bis praktisch unlösliche Verbindungen, insbesondere sehr schwer lösliche bis praktisch unlösliche Verbindungen, bevorzugt.

"Sehr schwer löslich" entsprechend Klasse 6 kann durch folgende Versuchsbeschreibung veranschaulicht werden:
Ein Gramm des zu untersuchenden Polyglucans werden in 1 I entionisierten Wasser auf 130° C unter einem Druck von 1 bar erhitzt. Die entstehende Lösung bleibt nur kurzzeitig über wenige Minuten stabil. Beim Erkalten unter Normalbedingungen fällt die Substanz wieder aus. Nach Abkühlung auf Raumtemperatur und Abtrennung mittels Zentrifugation können unter Berücksichtigung der experimentellen Verluste mindestens 66 % der eingesetzten Menge zurückgewonnen werden.

Die erfindungsgemäß eingesetzten Poly-α-glucane können beliebigen Ursprungs sein, solange die vorstehend angegebenen Bedingungen in bezug auf die Begriffe "linear" und "wasserunlöslich" erfüllt sind.

Sie können natürlich oder insbesondere auf biotechnischen Wege gewonnen sein.

Beispielsweise können sie aus natürlichen pflanzlichen oder tierischen Quellen durch Isolierung und/oder Aufreinigung erhalten werden.

Es können auch Quellen zum Einsatz kommen, die gentechnisch derart manipuliert worden sind, daß sie im Vergleich zu der unmanipulierten Quelle einen höheren Anteil an nicht oder vergleichsweise geringfügig verzweigten Polyglucanen enthalten.

Sie können auch durch enzymatische oder chemische Entzweigung aus nichtlinearen Polyglucanen hergestellt worden sein.
Dabei können nicht-lineare Polyglucane, die Verzweigungen enthalten, derart mit einem Enzym behandelt werden, daß es zur Spaltung der Verzweigungen kommt, so daß nach ihrer Abtrennung lineare Polyglucane vorliegen. Bei diesen Enzymen kann es sich beispielsweise um Amylasen, iso-Amylasen, Gluconohydrolasen, Cyclomaltodextrin-glucanotransferasen oder Pullulanasen handeln.

Biotechnische Methoden umfassen biokatalytische, auch biotransformatorische, oder fermentative Prozesse.

Lineare Poly-α-glucane hergestellt durch Biokatalyse (auch: Biotransformation) im Rahmen dieser Erfindung bedeutet, daß das lineare Polyglucan durch katalytische Reaktion von monomeren Grundbausteinen wie oligomeren Sacchariden, z.B. von Mono- und/oder Disacchariden, hergestellt wird, indem ein sogenannter Biokatalysator, üblicherweise ein Enzym, unter geeigneten Bedingungen verwendet wird. Man spricht in diesem Zusammenhang auch von "in vitro Biokatalyse".

Linerare Polyglucane aus Fermentationen sind im Sprachgebrauch der Erfindung lineare Polyglucane, die durch fermentative Prozesse unter der Verwendung in der Natur vorkommende Organismen, wie Pilzen, Algen, Bazillen, Bakterien oder Protisten oder unter der Verwendung von in der Natur nicht vorkommender Organismen, aber unter Zuhilfenahme von gentechnischen Methoden allgemeiner Definition modifizierten natürlichen Organismen, wie Pilzen, Algen, Bazillen, Bakterien oder Protisten gewonnen werden oder unter Einschaltung und Mithilfe von fermentativen Prozessen gewonnen werden können. Man spricht in diesem Zusammenhang auch von "in vivo Biokatalyse".

Beispiele für derartige Mikroorganismen sind Piichia pastoris, Trichoderma Reseii, Straphylokkus Camosus, Escherichia Coli und Aspergillus Niger

Vorteilhafte Verfahren für die biotechnische Gewinnung sind z. B. in der WO 95/31553 oder der nicht vorveröffentlichten deutschen Patentanmeldung der Anmelderin mit amtlichen Aktenzeichen 198 27 978.5 beschrieben.

Gemäß der WO 95/31553 werden Amylosucrasen zur Herstellung von linearen Poly-α-glucanen wie Poly-1,4-α-D-glucan mittels eines biokatalytischen Prozesses verwendet. Weitere geeignete Enzyme sind Polysaccharidsynthasen, Stärkrsynthasen, Glycoltransferasen, 1,4-α-D-Glucantransferasen, Glycogensynthasen oder auch Phosphorylasen.

Es können auch modifizierte wasserunlösliche lineare Poly-α-glucane eingesetzt werden, wobei die Poly-α-glucane beispielsweise durch Veresterung und/oder Veretherung in einer oder mehreren nicht an der linearen Verknüpfung beteiligten Positionen chemisch modifiziert worden sein können. Im Fall der bevorzugten 1,4 verknüpften Poly-α-glucane kann die Modifizierung in 2-, 3- und/oder 6-Position erfolgen. -

Modifikation im Sinne der Erfindung bedeutet, daß die vorhandenen Hydroxylgruppen, die nicht an der Verknüpfung beteiligt sind, chemisch verändert werden. Dies schließt eine Ringöffnung der Glucaneinheiten aus wie sie z.B. bei der oxidativen Carboxylierung oder der Hydrolyse erfolgt. Maßnahmen für derartige Modifizierungen sind dem Fachmann hinlänglich bekannt.

So können lineare Polyglucane wie z.B. Pullulane, die an sich wasserlöslich sind, durch Modifizierung wasserunlöslich gemacht werden.
Für die vorliegende Erfindung werden bevorzugt wasserunlösliche lineare Polyglucane eingesetzt, die in einem biotechnischen, insbesondere in einem biokatalytischen oder einem fermentativen, Prozeß hergestellt worden sind, wobei biokatalytisch hergestelltes Poly-α-glucan besonders bevorzugt ist.

Im Gegensatz zu Poly-α-glucanen, die aus natürlichen Quellen, wie Pflanzen, isoliert werden, weisen die hierbei erhaltenen linearen wasserunlöslichen Polyglucane ein besonders homogenes Eigenschaftsprofil auf, z. B. in bezug auf die Molekulargewichtsverteilung, sie enthalten keine oder allenfalls nur in sehr geringen Mengen unerwünschte Nebenprodukte, die aufwendig abgetrennt werden müssen oder zu allergenen Reaktionen führen könnten, und lassen sich exakt spezifiziert auf einfache Weise reproduzieren.

Zwar können auch mit der chemischen oder enzymatischen Entzweigung vergleichsweise homogene Produkte erhalten werden. Jedoch verbleibt in vielen Fällen ein Rest an nicht oder nur unzureichend entzweigten Ausgangsmaterial, das nur schwer abgetrennt werden kann.

Biotechnische und insbesondere biokatalytische Methoden haben den Vorteil, daß direkt wasserunlösliche lineare Poly-α-glucane erhalten werden können, wie z. B. die bevorzugten Poly-1,4-α-D-glucane, die keine Verzweigungen enthalten, bzw. deren Verzweigungsgrad unterhalb der Nachweisgrenze herkömmlicher analytischer Methoden liegt.

Die Poly-α-glucane können in Form sogenannter alpha-amylaseresistenter Poly-α-glucane eingesetzt werden wie sie am Beispiel von Poly1,4-α-D-glucan in der nicht vorveröffentlichten deutschen Patentanmeldung mit amtlichen Aktenzeichen 198 30 618.0 der Anmelderin beschrieben sind.

Alpha-amylaseresistente Poly-α-glucane können durch Herstellung einer Suspension oder Dispersion aus wasserunlöslichen Polyglucanen und Wasser, Erwärmen der Suspension oder Dispersion auf eine Temperatur im Bereich von 50 bis 100 °C, Abkühlenlassen der erhaltenen kleisterartigen Mischung auf eine Temperatur im Bereich von 50 °C bis an den Gefrierpunkt, vorzugsweise 35 bis 15 °C, 27 bis 22 °C, 16 bis 0 °C oder 6 bis 2°C, über einen Zeitraum von 1 bis 72 h, vorzugsweise 1 bis 36 h und insbesondere 15 bis 30 h und Retrogradation der kleisterartigen Mischung bei einer gegenüber der Temperatur der erwärmten kleisterartigen Mischung emiedrigten Temperatur in einem Temperaturbereich von 90 bis 4 °C sowie gegebenenfalls Trocknung oder Entwässerung des erhaltenen Produktes erhaltenen werden.

Das Poly-α-glucan kann auch als thermoplastisches Polyglucan eingesetzt werden, das erhältlich ist durch Aufschmelzen von linearem wasserunlöslichen Polyglucan und Hinzufügen von mindestens 20 Gew.%, vorzugsweise mindestens 30 Gew.%, eines Weichmachers wie Sorbitol, Glycerin, deren Kondensationsprodukte und Oligomere, DMSO, Bernsteinsäure, Citronensäure-Monohydrat, Apfelsäure, Weinsäure etc. bei ca. 170 °C.
Eine Beschreibung von geeigneten Maßnahmen und Eigenschaften von thermoplastischen Polyglucanen am Beispiel des bevorzugten linearen wasserunlöslichen Poly1,4-α-D-glucans gibt die nicht vorveröffentlichte deutsche Patentanmeldung mit amtlichen Aktenzeichen 198 52 826, auf die hierfür ausdrücklich bezug genommen wird.
Zur Verbesserung der Einmischbarkeit kann das thermoplastische Poly-α-glucan vorab in bekannterweise granuliert werden.

Die Molekulargewichte M_{w} (Gewichtsmittel, bestimmt mittels Gelpermeationschromatographie im Vergleich zu einer Eichung mit Pullulanstandard) der erfindungsgemäß verwendeten wasserunlöslichen linearen Poly-α-glucane können in einem weiten Bereich von 0,75 x 10² g/mol bis 10⁷ g/mol variieren. Bevorzugt liegt das Molekulargewicht M_{w} in einem Bereich von 10³ g/mol bis 10⁶ g/mol und besonders bevorzugt von 10³ g/mol bis 10⁵ g/mol. Ein weiterer vorteilhafter Bereich ist von 2 x 10³ bis 8 x 10³. Entsprechende Bereiche gelten für das bevorzugt eingesetzte Poly-1,4-α-D-glucan.

Die Molekulargewichtsverteilung bzw. Polydispersität M_{w}/Mₙ kann ebenfalls in weiten Bereichen je nach Herstellungsverfahren des Polyglucans variieren. Bevorzugte Werte sind von 1,01 bis 50, insbesondere von 1,01 bis 15, wobei kleine Polydispersitätswerte besonders bevorzugt sind, z.B. von 1,01 bis 2,5.

Dabei nimmt die Polydispersität mit einer bimodalen Verteilung der Molekulargewichte zu.

Zur Herstellung der Sonnenschutzmittel oder anderen Zubereitungen kann ein einziges wasserunlösliches lineares Poly-α-glucan oder eine Mischung aus zwei oder mehreren davon eingesetzt werden.

Aufgrund ihrer Naturidentität kann für die erfindungsgemäß eingesetzten wasserunlöslichen linearen Poly-α-glucane eine ausgezeichnete Biokompatibilität erwartet werden.

Nachstehend wird die vorliegende Erfindung anhand einzelner Beispiele veranschaulicht.

### Beispiel 1

In-vitro-Produktion von Poly-1,4-α-D-glucan in einem biokatalytischen Prozeß mit Hilfe von Amylosucrase.

In einem sterilisierten (Dampfsterilisation) 15 l Gefäß werden 10 l einer 20%igen Saccharose-Lösung gegeben. Der Enzymextrakt, Amylosucrase enthaltend, wird in einer Portion zugegeben. Die Enzymaktivität beträgt in diesem Experiment 16 units. Die Apparatur wird mit einem ebenfalls sterilisierten KPG-Rührer versehen. Das Gefäß wird verschlossen und bei 37°C aufbewahrt und gerührt. Bereits nach einer Zeit von wenigen Stunden bildet sich ein weißer Niederschlag. Die Reaktion wird nach einer Zeitdauer von 180 Stunden beendet. Der Niederschlag wird abfiltriert und zur Abtrennung niedermolekularer Zucker fünf Mal mit Wasser gewaschen. Der im Filter verbleibende Rückstand wird bei 40°C im Trockenschrank unter Anlegung eines Vakuums mit Hilfe einer Membranpumpe (Firma Vacuubrand GmbH & Co, CVC 2) getrocknet. Die Masse beträgt 685 g (Ausbeute 69 %).

### Beispiel 2

Charakterisierung des mit Amylosucrase synthetisierten wasserunlöslichen linearen Poly-1,4-α-D-glucans aus Beispiel 1

Es werden 2 mg des Poly-1,4-α-D-glucans aus Beispiel 1 bei Raumtemperatur in Dimethylsulfoxid (DMSO, p.a. von Riedel-de-Haen) gelöst und filtriert (2 µm Filter).

Ein Teil der Lösung wird in eine Gelpermeationschromatographie Säule injiziert. Als Elutionsmittel wird DMSO verwendet. Die Signalintensität wird mittels eines RI-Detektors gemessen und gegen Pullulanstandards (Firma Polymer Standard Systems) ausgewertet. Die Flußrate beträgt 1.0 ml pro Minute.

Die Messung ergibt ein Zahlenmittel des Molekulargewichts (Mₙ) von 14.200 g/mol und ein Gewichtsmittel des Molekulargewichts (M_{w}) von 29.500 g/mol. Dies entspricht einer Dispersität von 2,1.

### Beispiel 3

Bewertung des Poly-1,4-α-D-glucans nach Beispiel 1 als UV-Filter

Die Bewertung erfolgte nach den Vorschriften der COLIPA Sun Protection Factor Test-Methode.

Für die Untersuchung wurden Versuchspersonen ausgewählt, welche in der Verteilung ihrer UV-Empfindlichkeit der Anwendermehrzahl entsprachen, UVgewöhnte wurden ausgeschlossen.

Es wurde die Standardpräparate P1 und P3 (Fa. Beiersdorf AG) im Vergleich zu einer Zubereitung mit 5 Gew.% wasserunlöslichem linearen Poly-1,4-α-D-glucan nach Beispiel 1 verwendet.

Die wesentlichen Untersuchungsparameter waren:

Verwendung eines Sonnensimulators nach Schrader (SU 2000, Hersteller PTI-Photon Technology GmbH), der mit einer 300 Watt Xenon-Kurzbogenlampe ein Licht mit einem repräsentativen Spektrum erzeugt, das auf die Intensitätswerte von 320 nm normiiert war.

Der Lichtstrahl dieser Lampe wurde mit Hilfe von beweglichen Spiegeln auf sechs kreisförmig angeordnete Punkte gerichtet, so daß die homogene Bestrahlung mit 6 unterschiedlichen Lichtdosierungen innerhalb einer Sitzung möglich war.

Zur exakten Applikation der von der COLIPA-Norm geforderten Menge des Sonnenschutzmittels von 2,0 +/- 0,04 mg/cm² wurde das Sonnenschutzmittel mit einer Plastikspritze auf einen Kunststoffspatel aufgetragen und mit diesem auf auf der zu bestrahlenden Fläche gleichmäßig verteilt.

Die Ablesung der Bestrahlungsfelder erfolgte nach 20 Stunden +/- 4 Stunden.

Der mittlere Lichtschutzfaktor (LSF)wurde nach folgender Formel errechnet, worin MED für minimale erythemale Dosis steht:
LSF (COLIPA) = MED-Testfeld / MED-Leerfeld

Es wurde ein mittlerer Lichtschutzfaktor von 5,8 mit einer Standartabweichung von 0,7 erhalten.
Das Vertrauensintervall (V95%) lag innerhalb 20 % des Mittelwertes.

## Patentansprüche

1. Verwendung von wasserunlöslichen Poly-α-glucan mit einem Verzweigungsgrad in 6-Position von kleiner gleich 0,5% als UV-Filter.

2. Verwendung nach Anspruch 1, **dadurch gekennzeichnet, daß** das Poly-α-glucan Poly-1,4-α-D-glucan ist.

3. Verwendung nach einem der vorhergehenden Ansprüche, **dadurch gekennzeichnet, daß** das Poly-α-glucan in einer Menge von etwa 0,5 bis etwa 70 Gew. % eingesetzt wird, bezogen auf das Gesamtgewicht der einen UV-Filter enthaltenden Zubereitung.

4. Verwendung nach einem der vorhergehenden Ansprüche, **dadurch gekennzeichnet, daß** das wasserunlösliche lineare Poly-α-glucan biotechnisch, insbesondere biokatalytisch, erzeugt worden ist.

5. Verwendung nach einem der vorhergehenden Ansprüche in einem kosmetischen Mittel wie Körperpflegemittel oder einer dekorativen Kosmetik.

6. Verwendung nach Anspruch 5, **dadurch gekennzeichnet, daß** das kosmetische Mittel ausgewählt ist unter Cremes, Kompaktcremes, Lotionen, Milchen, Masken, Puder, Salben und Salbengrundlagen.

7. Verwendung nach Anspruch 6, **dadurch gekennzeichnet, daß** die dekorative Kosmetik ausgewählt ist unter Cremes, Puder oder Fonds für Make-up.

8. Verwendung nach einem der vorhergehenden Ansprüche zur Herstellung eines Sonnenschutzmittels.

9. Sonnenschutzmittel enthaltend mindestens ein biotechnisch erhaltenes, wasserunlösliches lineares Poly-α-Glucan mit einem Verzweigungsgrad in 6-Position von kleiner gleich 0,5 % als UV-Filter.

## Claims

1. The use of water-insoluble poly-α-glucan having a degree of branching of less than or equal to 0.5% as UV filter.

2. The use as claimed in claim 1, wherein the poly-α-glucan is poly-1,4-α-D-glucan.

3. The use as claimed in any of the preceding claims, wherein the poly-α-glucan is used in an amount of from about 0.5 to about 70% by weight, based on the total weight of the preparation comprising a UV filter.

4. The use as claimed in any of the preceding claims, wherein the water-insoluble linear poly-α-glucan has been produced biotechnologically, in particular biocatalytically.

5. The use as claimed in any of the preceding claims in a cosmetic product, such as bodycare product or a decorative cosmetic.

6. The use as claimed in claim 5, wherein the cosmetic product is chosen from creams, compact creams, lotions, milks, masks, powders, ointments and ointment bases.

7. The use as claimed in claim 6, wherein the decorative cosmetic is chosen from creams, powders or bases for make-up.

8. The use as claimed in any of the preceding claims for the preparation of a sun protection product.

9. A sun protection product comprising at least one biotechnologically obtained, water-insoluble linear poly-α-glucan as UV filter.

## Revendications

1. Utilisation de poly-α-glucane insoluble dans l'eau avec un degré de ramification en position 6 inférieur ou égal à 0,5 % comme filtre UV.

2. Utilisation selon la revendication 1, **caractérisée en ce que** le poly-α-glucane est le poly-1,4-α-D-glucane.

3. Utilisation selon l'une des revendications précédentes, **caractérisée en ce que** l'on utilise le poly-α-glucane en une quantité d'environ 0,5 à environ 70 % en poids, par rapport au poids total de la préparation contenant un filtre UV.

4. Utilisation selon l'une des revendications précédentes, **caractérisée en ce que** le poly-α-glucane linéaire insoluble dans l'eau est généré par voie biotechnologique, en particulier biocatalytique.

5. Utilisation selon l'une des revendications précédentes dans un agent cosmétique comme agent de soin corporel ou cosmétique décoratif.

6. Utilisation selon la revendication 5, **caractérisée en ce que** l'agent cosmétique est choisi parmi les crèmes, les crèmes compactes, les lotions, les laits, les masques, les poudres, les onguents et les bases d'onguents.

7. Utilisation selon la revendication 6, **caractérisée en ce que** l'on choisit le cosmétique décoratif parmi les crèmes, les poudres ou des fonds de fard.

8. Utilisation selon l'une des revendications précédentes pour la préparation d'un produit anti-solaire.

9. Produit anti-solaire contenant en position 6 au moins un poly-α-glucane linéaire insoluble dans l'eau, obtenu par voie biotechnologique, ayant un degré de ramification inférieur ou égal à 0,5 % comme filtre UV.
